# EUROPEAN PATENT APPLICATION

(11) **EP 2 469 433 A1**
(43) Date of publication of application: **27.06.2012**
(21) Application number: 10196210.8
(22) Date of filing: 21.12.2010
(51) Int. Cl.: G06F 19/00

(54) **Therapeutic/logistic managing system**

(71) Applicant: Gvm Ideando S.r.l., 48022 Lugo (RA) (IT)
(72) Inventor: Ceccarelli, Riccardo, 56122, Pisa (IT)
(74) Representative: Postiglione, Ferruccio

(57) **Abstract**

It is described a logistic/therapeutic management system comprising: at least one management computer (WKS1) provided with at least one management software module operating on a distributed database (DB-3) containing data associated to drugs/medical-surgical devices and to patients of one or more Sanitary Institutions of a more or less complex Sanitary Organization. The object is to manage at least one cabinet/trolley (200B) adapted to receive drugs and/or medical-surgical devices. Moreover, the system comprises user interface means (10, 11, 14, 15) of a modular-type adapted to exchange information/control signals with the at least one cabinet/trolley and technical interface means (8, 9, 12, 13) adapted to manage the communications and the closure of the cabinets between the at least one management computer (WKS1) and the at least one cabinet/trolley

## Description

### Field of the invention

The present invention refers to the field of the management systems and, particularly, to the logistic/therapeutic management in hospital departments.

### Prior art

The logistic/therapeutic management systems present on the market can be classified in decentralized-type systems and centralized-type systems.

The decentralized-type approach provides the manufacture of electronic cabinets/trolleys for substituting the existing conventional ones, with a consequent substantial structural impact.

The potential investment in conventional cabinets/trolleys is therefore lost because it is necessary to substitute them with electronic cabinets/trolleys and to transfer the sanitary products in the latter.

Instead, the approach known as centralized, provides a robotic apparatus located in the Hospital Central Pharmacy capable of preparing personalized medications for the patients with the following necessity to increase the logistic/distributing flow from the Central Pharmacy to the departments, in order to distribute the medications to the patient bedside. This approach causes critical situations especially in Hospital Institutions structured in more departments.

Other approaches propose a logistic/therapeutic management of the software-type but, for their nature, they do not have a physical control on the sanitary products.

### Summary of the invention

The Applicant has observed that the logistic/therapeutic management systems according to the prior art are not very versatile and are sometimes invasive in that they often require to radically change the way the operators of an hospital department and/or of the organizing chain located upstream the department (Central Depositories: Supply Depository, Pharmacy Depository) work, in order to guarantee the availability of the sanitary products necessary to treat the patients.

Moreover, since the types of cabinets and trolleys commercially available and present in the sanitary institutions are very different by size/shape/material (they are not standardized), it exists a following constraint and/or chance to find a solution adaptable to any field.

The problem underlying the present invention is related to the observation that a complete and versatile system for managing the logistics at hospital departments, allowing, for example, to preserve the existing cabinets and trolleys, is a necessity that presently is not fulfilled by the market.

The present invention refers to a logistic/therapeutic management system as defined in the attached claim 1 and to its preferred embodiments described in the depending claims 2-16.

### Brief description of the drawings

Other characteristics and advantages of the invention will be better understood by the following description of a preferred embodiment and of its variants, illustratively provided referring to the attached drawings, wherein:
- Figure 1 shows an illustrative diagram of a distributed database on which is based the logistic/therapeutic management system according to the invention;
- Figure 2 schematically shows an example of two Workstations having installed application components and a local database, some apparatuses and three cabinets/trolleys comprised in said logistic/therapeutic management system;
- Figure 3 shows an example of a display of a displaying module useable in said logistic/therapeutic management system;
- Figure 4 shows an example of "conditioned" cabinets according to a particular embodiment of said logistic/therapeutic management systems;
- Figures 5A, 5B and 5C show a particular embodiment of a displaying module, a selection module, and a closing module, respectively, applicable to cabinets/trolleys of the logistic/therapeutic management system;
- Figure 6 shows a manual unlocking device.

### Detailed description of the invention

### Architectural and structural description of a particular embodiment.

There will be described some examples of embodiment of a logistic/therapeutic management system of a department for complex sanitary institutions such as, for example, Local Sanitary Enterprises, Hospital Enterprises, groups of Sanitary Institutions, etc.

Particularly, the management system offers the possibility to adapt to different hierarchical organizational architectures and provides, in the most complex case, a central level (Local Sanitary Enterprises, Hospital Enterprises, groups of Sanitary Institutions, etc.), an intermediate level (Sanitary Institution, Hospital Facility, General Outpatients Departments, etc.) and a base level (Operative Units with their possible sub-organizations).

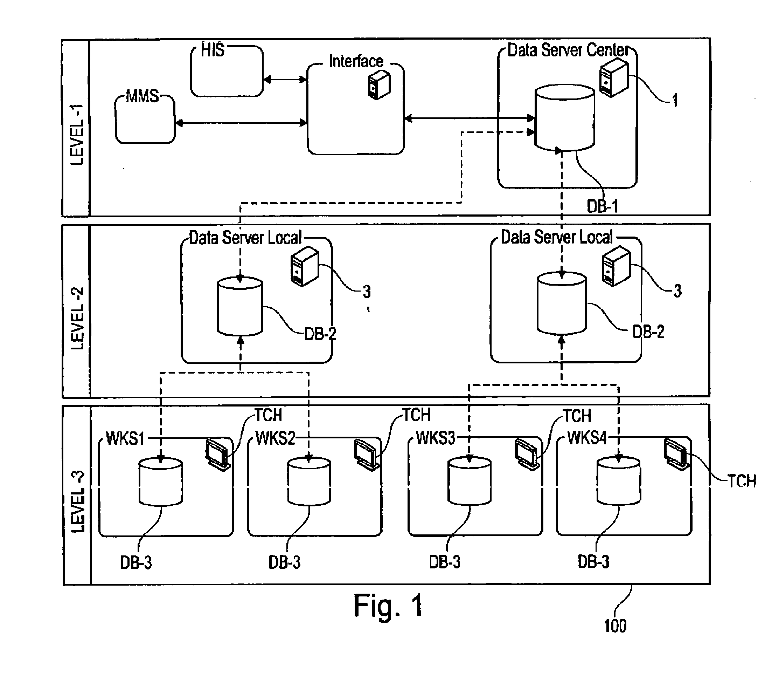

Referring particularly to the particular example of Figure 1, wherein it is shown the architecture 100 of a distributed database on which is based the management system and that provides the database organized on three levels. According to Figure 1, the distributed database is configured on three levels:
Level-1: Central Database of the Organization (DB-1);
Level-2: Local Database of an Hospital Facility, General Outpatients Department, etc (DB-2).
Level-3: DB Local Workstation (DB-3).

It is to be noted that the number of levels can differ according to the system arrangement.

More specifically (referring always to Figure 1), the architecture 100 comprises a DB central server 1, such as a Central Data Server, wherein there is located a central database, DB-1 of the whole organization; the central database DB-1 is synchronized with data coming from local databases DB-2 belonging to the level Level-2.

Moreover, the architecture 100 comprises an interface server 2, adapted to store interface programs of the software system 100 with software of third parties, usually present in the hospital fields such as a HIS (Hospital Information System) software and a logistic administrative software MMS (Material Management System), etc.

The architecture 100 is also provided with:
- one or more DB local servers 3, such as local data servers, containing corresponding local databases DB-2 relating to single sanitary institutions. The local databases DB-2 are synchronized with the data of the database DB-3 of the Workstations WKS1-WKS4, belonging to the Level-3, and with the data sent/received to/from the DB central server 1, from the database DB-1.

The Workstations WKS1 - WKS4 (for example, each of them is associated to a different room of the hospital institution), are for example "all in one" personal computers, preferably provided with touch screens TCH and containing all the computational hardware and connected to the computer network of the hospital (LAN); said personal computers (WKS1-WKS4) are dedicated to the management of cabinets/trolleys.

The central database DB-1 and/or the local databases DB-2 and DB-3 store, among other things, data associated to patients, drugs, medical-surgical devices, medications, etc. and to drugs/medical-surgical devices to be administered/distributed/implanted in patients in relation to a corresponding therapeutic medication or procedural sanitary obligations.

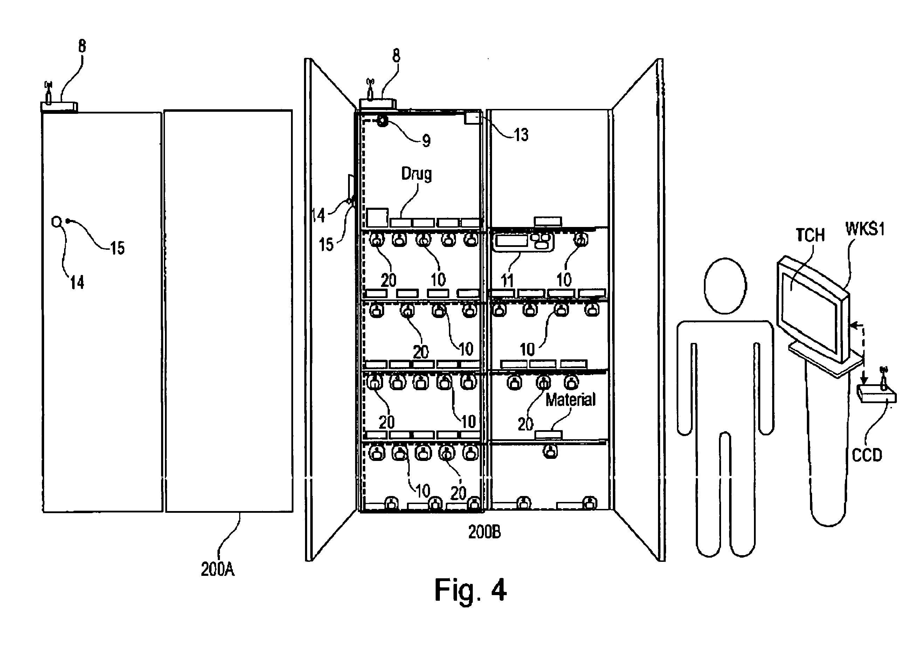

To this end, it is to be observed that the described cabinets/trolleys. The term "cabinet/trolley" means a real cabinet and/or trolley. Particularly, a cabinet can be seen as a piece of furniture comprising shelves and/or drawers, and possibly closed by at least one door (Figure 4), adapted to contain drugs or medical-surgical devices according to an ordered arrangement organized by locations (shelves), positions (partitioning of a location in more sections) and depth (partitioning of a section in more parts), in order to allocate a sanitary product according to a three-dimensional cell arrangement.

The term "trolley" means a wheeled transportable support surface and/or a container (preferably provided with at least one door) adapted to contain drugs or medical-surgical devices according to an ordered organized arrangement as explained before with reference to the cabinet. Such trolleys can also be employed by the users (typically paramedics) for administrating/distributing of the prescribed drug medications at the bedside of patients.

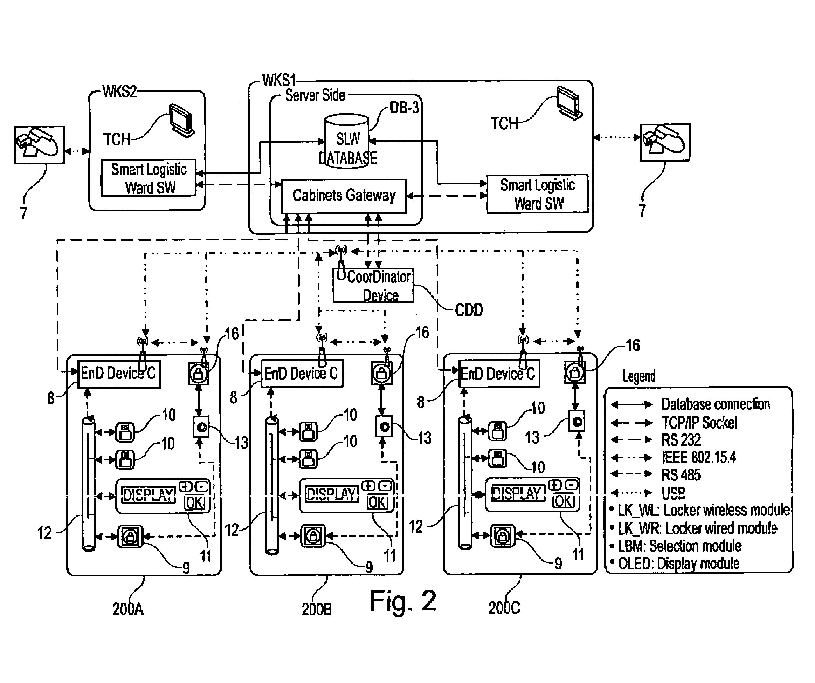

Referring also to Figure 2, it is shown the example of two Workstations WKS1 and WKS2, present in the same room. Each Workstation stores a department management application software, called "Smart Logistic Ward", only one of the two Workstations stores the local database called DB-3 (Figure 1) and a software to communicate to the cabinets/trolleys, called "Cabinet Gateway".

The same approach can be employed also for more than two Workstations located in the same room, so that all the Workstations store the "Smart Logistic Ward" software while only Workstation stores the local database DB-3 and the communications software "Cabinet Gateway". Besides some apparatuses or software modules shown in Figure 1, Figure 2 schematically illustrates also three cabinets/trolleys 200A, 200B, 200C and two reading devices 7. For example, each of the reading devices 7 is such to enable the reading of bar codes and it is connected to one of the Workstations WKS1-WKS2 for obtaining both the simplification of the user login procedure to the system by reading a bar code printed on an user identifying badge and containing coded authentication credentials to the system, and the simplification of the sanitary product selection (drug or medical-surgical device) by reading the bar code of the product.

The management system comprises modular-type interface means adapted to exchange information/control signals between and user operating on a cabinet/trolley and the DB-3, local database of the Workstation.

The modular-type interface means are such to comprise at least two separated devices and each device being configured to deliver, with reference to the same cabinet/trolley, a different interface function.

Such interface means can comprise devices and apparatuses fitted on each cabinet/trolley 200A, 200B and 200C. According to another embodiment, the interface means are not fitted on the cabinet/trolley ("non-conditioned" cabinet/trolley) and the latter is directly managed by the "Smart Logistic Ward" software of the Workstations (WKS1-WKS4) present in the room receiving the cabinet/trolley.

The described management system is structured in order to communicate to the user, by data stored in the databases DB-1, DB-2, DB-3, to the "Smart Logistic Ward" software and to the interface means, a plurality of dosage/administration/distribution data and data identifying drugs/medical-surgical devices to be taken from the trolleys/cabinets 200A, 200B and 200C for a predetermined patient. The described management system moreover enables to store in the DB-Servers 1, 3 and in the Workstations (WKS1-WKS4), a plurality of data related to a sanitary product taken from a cabinet/trolley for a predetermined patient, thanks to the interface devices and to the management software, "Smart Logistic Ward".

Particularly, the interface means comprise a communications device 8 (End-device, EDD) enabling the communications between the cabinet/trolley 200A, 200B or 200C and the Workstations WKS1 and WKS2 influencing the DB-3 which, in turn, exchanges data with the DB-2 and the latter with the DB-1.

Such communications device 8 is fitted on each cabinet 200A, 200B and 200C. This apparatus enables the wired or the wireless communications with the Workstations WKS1 and WKS2 passing through the "Cabinet Gateway". Moreover, the communications means can comprise a coordinator, CDD, which can communicate in wireless, zigbee modes to the Workstations WKS1 - WKS2.

According to specific examples of the embodiment, the communications device 8 can communicate to the Workstations WKS1 and WKS2 by the cited software "Cabinet Gateway":
- in wireless, zigbee mode (IEEE 802.15.4);
- in wired mode, through Ethernet.

The communications device 8 can moreover comprise a serial wired communications RS485 enabling the communications with modular interface apparatuses fitted on the cabinet/trolley 200A, 200B and 200C.

According to an example, the modular interface apparatuses to be used for conditioning a cabinet/trolley can comprise one or more modules such as, particularly: a wired "closing" module 9 and/or a wireless closing module 16 (as shown on the cabinets/trolleys 200A, 200B, 200C in Figure 2); one or more "product selection" modules 10 and, preferably, a "displaying" module 11.

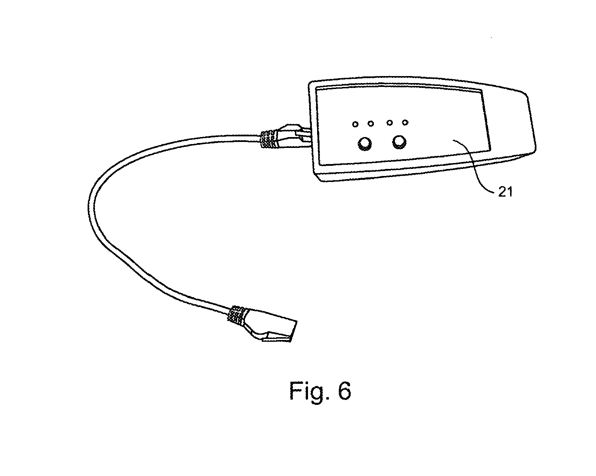

The closing module 9 is adapted to communicate (for example by the interface RS485) to the communications device 8 and an electrolock 13 (Figure 4 and Figure 2), arranged for inhibiting or enabling the opening/closure of a door of the cabinet/trolley 200A, 200B and 200C. The closing module 9, besides receiving a pulse upon opening/closing a Workstation, by the communications device 8 (EDD) of Figures 2 and 4, or directly by the wireless closing module 16, can receive it also by an unlocking key illustratively shown in Figure 6, inserted in a suitable lock 13, provided in the cabinet/trolley as shown in Figure 4.

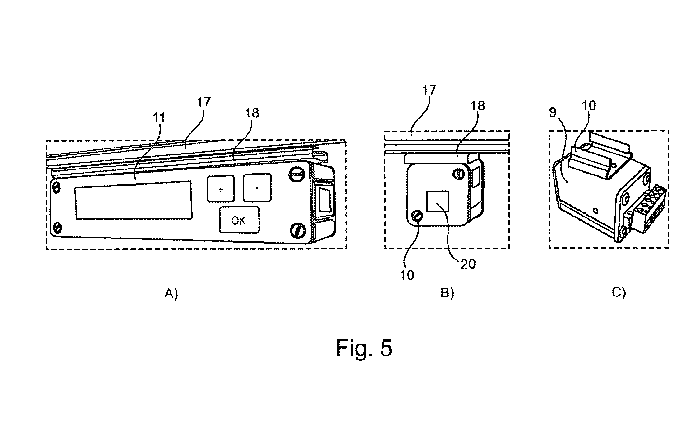

The "product selection" module 10 comprises a selection push-button 20 (Figure 5B) and an indicator consisting in an identifying/signalling LED. The selection push-button 20 enables the user to identify which product, drug or medical-surgical device, received on the upper portion of the shelf, should be used. Such selection module 10 can serially communicate, RS485, or can communicate in wireless, zigbee modes, by using piezoelectric push-buttons, to the communications device 8 (EDD) for reaching then, by the EDD 8 itself, the Workstations by the tcp/ip or zigbee protocol.

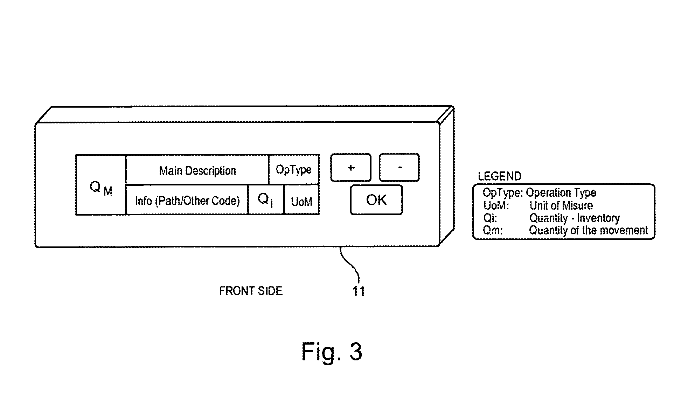

The "displaying" module 11 (Figure 3) is a displaying module locatable in the cabinet/trolley (Figure 4 and Figure 2) communicating to the EDD 8 by a serial connection, RS485, or zigbee mode, and then communicating by the EDD 8 itself with the room Workstation/s, through the tcp/ip or zigbee protocol.

The "display" module 11 is for example provided with quantity-modifying push-buttons "+" and "-" and a confirmation push-button "OK". The "display" can be implemented by the OLED technology, and a particular embodiment of it will be described in the following. According to the example of Figure 2, it is moreover provided a wired communications bus 12, particularly of the seven-lines bidirectional full duplex serial RS485 type, having an handshake line, inside each cabinet/trolley 200A, 200B and 200C and a supply module comprising a back-up battery (not shown) located on each cabinet/trolley. Such communications bus 12, as described before, can be used for connecting the closing module 9, the selection modules 10 and the displaying module 11 to the communications device 8. Particularly, the selection, displaying, closing modules 10, 11 and 9 are provided with an unique identifying hardware and a reset mechanism.

The cabinets/trolleys can provide another type of closing module, such as the 16 in Figure 2, of the wireless zigbee type and capable of directly communicating by the coordinator CDD besides the EDD 8. The communications device 8 is moreover provided with a configuration Deep-switch, for its addressing.

According to a particular embodiment, the coordinator CDD enables the set-up of the wireless zigbee network (IEEE 802.15.4) with a star shape, wherein the coordinator CDD is the start centre and the communications devices 8 are addressed by a "fully acknowledgement with packet integrity control" protocol. For example, the communications between the coordinator CDD and the Workstation WKS1 - WKS4 can be serial, by AT commands and binary structures on an asynchronous serial channel UART RS232 or by socket, protocol TCP/IP, on Ethernet. The coordinating module CDD is also provided with a configuration Dip-switch for its addressing.

The wireless zigbee network (IEEE 802.15.4) enables the communications between the coordinating module CDD (connected to the Workstation WKS1, WKS2) and the communications devices 8 located on the cabinets/trolleys or directly to any wireless closing modules 16.

According to the considered example, the communications between the cabinets/trolleys 200A, 200B and 200C and the corresponding Workstations WKS1, WKS2 is by an exchange of asynchronous messages, with a possible ack-nak request. All the communications coming from the electronic devices of the cabinets/trolleys 200A, 200B and 200C have a sender header with an identifying code and the device type (check device). For optimizing the message exchange, the system uses broadcast messages having a coding mask for simultaneously controlling several devices.

With reference to Figure 4, it is illustratively shown the cabinet 200B having two vertical compartments and four shelves. In the left-hand compartment (in Figure 4) there are five sanitary products on the upper shelf, four on the second shelf, five on the third, five on the fourth and three on the fifth shelf. Each shelf has labels showing a readable code, the description and the bar code of the sanitary products put in the shelf section at the back. The sanitary products can be drugs or medical-surgical devices. In the right-hand compartment, there are instead, starting from the top, respectively, a product on the first shelf, four sanitary products on the second one, three on the third, one on the fourth and two on the fifth shelf. At each product (drug or medical-surgical device), a plurality of selection modules 10 are for example fitted on the shelf, under or laterally the above-mentioned labels, these modules enable the user to declare which product (drug or medical-surgical device) is object of withdrawal, refill, inventory or return.

Still referring to Figure 4, on the cabinet 200A there are also schematically shown the electro lock 14 and a LED 15 signalling the open/closed condition of the door.

### Description of examples of usable software modules

Now, it will be made reference to the software modules which can be illustratively installed in the Workstations WKS1, WKS2, WKS3, WKS4.

The "Smart Logistic Ward" module manages the operability of the department Workstation WKS1-WKS4 and can communicate to the local database DB-3, with the Sanitary Institution database DB-2 or to the central database DB-1 of the Organization by an ODBC connection, while it communicates to the "Cabinet Gateway" by the TCP/IP protocol, for interacting with the cabinets/trolleys. The "Cabinet Gateway" is activated only by one of the Workstations present in a room.

The "Smart Logistic Ward" (Figure 2) has been developed, for example, with a smart client technology, and it provides a multilayer architecture organized by four levels: an user interface layer, a logic layer, a business layer, a data access layer. The "Smart Logistic Ward" is the main software module by which all the other modules are controlled.

Said module "Cabinet Gateway" enables the communications between the Workstations WKS1- WKS4 and the cabinets/trolleys 200 A-C, both by a serial RS232 bus with the coordinator CDD and between the latter and the EDDs 8 through zigbee, and by Ethernet, the TCP/IP protocol, directly to the EDDs. The software can manage the requests coming from several "Smart Logistic Ward" clients, therefore from several Workstations.

"DB Syncro" instead implements the synchronizing software process between the local database DB-3, in the Workstations WKS1 - WKS4 and the database DB-2, of the sanitary institution and between the latter and the central database, DB-1, of the Organization. It can be integrated by management systems of the replications of RDBMSs used for implementing the system.

The "Therapy manager" module is the therapeutic prescription software module.

The "Vital parameters manager" module is a software module enabling to manage the vital patient parameters. The "Electronic Patient Record" module is a software module enabling to manage the computerized chart.

The "Interface Manager" module enables the integration with other computerized systems, among which the HIS (Hospital Information System), for the synchronization with ADT (Admission, Demission & Transfer) and/or the possible Ambulatory Software module for obtaining personal data about the patients. Moreover, the "Interface Manager" module enables the integration with the MMS (Material Management System" for:
- obtaining the Hospital Therapeutic Handbook and the index of the Sanitary Institution, in other words the registry synchronization of the drugs and medical-surgical devices, with an information flow from the MMS to the "Interface Manager";
- automatically reorganizing the sanitary products, with an information flow coming from the "Interface Manager" to the MMS;
- unloading the used products on consignment for determined patients with an information flow from the "Interface Manager" to the MMS;
- delivering DDTs (Transport Documents) or documents relating the refilling of sanitary products, with an information flow from the MMS to the "Interface Manager".

### Operability examples of the described apparatuses/devices

There will be described now some illustrative hardware-type functionalities of the described logistic/therapeutic system, referring particularly to what has been described above and to the exemplifications shown in Figure 2 and Figure 4.

The system enables to open the cabinet/trolley by functionalities preset in the "Smart Logistic Ward" software.

In case of emergency/urgency, there is the possibility to unlock/open the doors of the cabinets/trolleys electronically controlled by a software routine monitoring the doors which are opened from time to time or by a manual unlocking key 21, Figure 6, connectable to the electrolock 14.

In case of a malfunction of the electrolock 14, it is possible to unlock it by a mechanical procedure.

It is moreover possible to optically signal the state of an cabinet/trolley: openable, open and closed, by turning on/off the LED 15 located on the corresponding cabinet/trolley 200A - 200C.

With reference to the drug or medical-surgical device selection, as it was said before, the selection of a product located in a specific section of the shelf is made possible by depressing a suitable push-button 20 on the product selection module 10, and optically signalling the selected product, by turning on the corresponding LED.

Moreover, it is possible to identify, by turning on the corresponding LEDs, the list of the sanitary products to be selected.

With reference to the displaying module 11 shown in Figure 3, it is to be observed that it enables to:
- show on a portion "Op Type" of the display the type of operation in progress in order to distinguish among a withdrawal "P", refilling "R" and inventory "I" operations;
- show on a corresponding portion Q_{M} of the display the amount of the selected product;
- show on a corresponding portion UoM of the display a measure of a product taken;
- show on a portion "Main description" the code and description of the selected product;
- show on a portion "info" of the display the code and the path, inside the cabinet/trolley, by the format "location.position.depth", intended to indicate the placement of a product; therefore, the path is shown by three coordinates: such as "the location" (for example, a, b, c, etc.), that is the corresponding shelf, with an order from the top to the bottom, "the position" (for example, 1, 2, 3, etc.) that is the section, from the left to the right, along the axis of the shelf and "the depth" (for example, k1, k2, k3, etc.), that is the subdivision of the shelf section in more depth levels starting from the outside to the inside of the cabinet/trolley;
- show on a portion Qi of the display the inventory amount of the selected product, that is the quantity available in the product placement;
- modify the amount of the product on which are performed the withdrawal/refill/return/inventory operations by depressing the push-buttons "+" or "-";
- optically signal the sanitary products, with withdrawal/return/refill quantities Q_{M}>0;
- modify, during the withdrawal/return/refill operations, the available inventory quantity Qᵢ by simultaneously depressing the push-buttons "+" and "-" and by correspondingly confirming it (OK).

Further, the described system enables to signal the inner state of the devices and to associate a logic address to each installed electronic device, during the start-up system step. There is also the possibility to reset the electronic devices by depressing the suitable reset push-button.

Figure 5 shows an example of embodiment of the displaying module 11 (Figure 5A), of the selection module 10 (Figure 5B) and of the closing module 9 (Figure 5C) implemented as apparatuses provided with means for removably fixing them to a cabinet/trolley. Particularly, according to Figure 5, the displaying module 11, the selection module 10 and the closing module 9 have tabs 18 adapted to engage a suitable support guide 17, mountable on a cabinet/trolley in a position suitable for the module function.

### Example of an use for a sanitary activity

Referring to a sanitary activity, the management software comprising, as described before, several modules, enables to operate on several logic levels, such as: the Central Organization (for example, Local Sanitary Enterprise, Group, etc.); the Sanitary Institution/Hospital Facility; the Operative Unit/Department - the Operative Sub-unit, in order to manage more or less complex hospital organizations.

In any case, the system enables the logistic/therapeutic management of any hospital department.

The smart Logistic Ward" software electronically tracks the operator access to the system, the performed activity, the patient involved in the activity (when the activity provides this), the product/products affected by the activities, the involved quantity and when (date and hour) was executed the operation.

Advantageously, the "Smart Logistic Ward" software supplies data for possible analyses associated to the operations performed on the system both at patient level and sanitary products and/or medication level.

The identification of the operator, that is the user (login step), can occur both by reading a bar code, printed on the user badge, comprising suitably crypted account and password (when there is available a bar code reader on the Workstation WKS1-WKS4), and by entering the account and password in suitable boxes.

The "Smart Logistic Ward" software verifies, for each activity, the authorization level of the identified operator.

In the following, there will be described some possible modes for managing activities withdrawing a product from a cabinet/trolley to be administered to or used for an hospitalized patient and/or an outpatient.

Withdrawal mode for "not conditioned" cabinets/trolleys

In case of not conditioned cabinets/trolleys, in other words not provided with software interface means, the room Workstation WKS1- WKS4 user executes first his/her identification with the system, then the selection of the patient, lastly, withdraws the medication or selects the necessary sanitary product list. It is possible to select a product by reading the corresponding bar code by the reader 7. Upon the confirmation, the system records the withdrawal operation.

### Withdrawal mode for cabinets/trolleys having a "light control"

In case of cabinets/trolleys with a light control, that is which are made "smart" just by the access control electronics, but not by an electronics allowing the selection and modification of data relating the withdrawals, the user, after having defined the withdrawal list, as described in the preceding mode, enables the opening of cabinets/trolleys (the sub-group of cabinets/trolleys containing the sanitary products selected, called Working Cabinet).

The cabinet/trolley opening system provides (considering, for example, the cabinet 200A associated to the Workstation WKS1):
- the optical signalling, by the LED 15, of the cabinet 200A involved in the operation;
- the opening of the cited cabinet 200A by the user;
- upon opening the cabinet 200A containing the sanitary products selected or being part of the medication, the user possibility of withdrawing them;
- upon closing the cabinet 200A, the LED 15 turns off (optical confirmation of the closed state) and the possibility of withdrawing the sanitary products contained in another still closed cabinet/trolley and being part of the Working Cabinet, for example the cabinet 200B if it contains one or more sanitary products selected or being part of the medication;
- that all the LEDs of the Working Cabinet are off at the end of the routine.

The user can confirm the withdrawal at the Workstation WKS1.

### Withdrawal mode for "medium control" cabinets/trolleys

This mode is enabled by a suitable configuration of the conditioned cabinets/trolleys, provided with closing modules 9 and displaying modules 11. It should be made reference, for example, to Figures 2, 3 and 4 without the selection modules 10.

The user, having confirmed the withdrawal list on the Workstation WKS1, can open the cabinets 200A and 200B, one after the other, as described in the preceding mode and the opening order is defined by the system as a function of an arrangement defined during the configuration step.

Upon opening the cabinet 200B, the system signals on the displaying module 11 that a withdrawal operation, "P", is in progress, shows the measure, the withdrawal amount and the Name and Code of the first Product to be taken from the cabinet 200B as a function of a specified placement by the path organized as described before: "position.location.depth".

A product after the other, the sanitary products, specified in the withdrawal list, and placed in the cabinet 200B, are shown on the displaying module, in this way the user is enabled to modify and confirm, one after the other, the predetermined withdrawal amounts for the preselected sanitary products, respectively by the push-buttons, +/- and OK, present on the displaying module 11.

As the amounts of the different preselected sanitary products of the cabinet 200B are confirmed (by the push-button OK of the displaying module 11), the symbol before the product code changes from "-" to "+", so that it identifies the sanitary products already confirmed and therefore taken.

At the end of the withdrawal from the cabinet 200B, the management system offers the possibility to open another cabinet/trolley containing other preselected sanitary products. The routine ends with the confirmation from the displaying module 11 of the sanitary products contained in the last cabinet/trolley involved in the withdrawal and with a final confirmation from the Workstation WKS2.

### Withdrawal mode for cabinets/trolleys having a "total control"

This mode is enabled by a suitable configuration on the conditioned cabinets/trolleys, each having the closing module 9, the displaying module 11 and a number of selection modules 10 equal to the number of the sanitary products types allocated in each cabinet/trolley.

The user, having confirmed the withdrawal list on the Workstation WKS2, can open the cabinets 200A and 200B, one after the other, as described in the preceding mode and the opening order is set by the system as a function of an arrangement defined in the configuration step. Upon opening the cabinet 200B, the system signals to the operator the preselected sanitary products (proposed list) from the Workstation WKS1, by turning on the LEDs of the selection modules 10 associated to the preselected sanitary products.

The displaying module 11 shows, from time to time, the withdrawal operation, "P", the administration measure, the withdrawal amount and the name and code of the preselected product, after having depressed the push-button 20 (Figure 5C) of the selection module 10, whose LED starts intermittently flashing (initially, upon opening the cabinet/trolley, the display is off).

The user, by selecting one of the preselected sanitary products, can modify the withdrawal amount by the push-buttons +/- on the displaying module and can confirm the amount by the push-button "OK".

With reference to Figure 5B, the system signals, by the green colour selection module 10 LED, all the sanitary products having a taken amount Q_{M} greater than 0 and with the red colour LED all the sanitary products of the proposed list not already confirmed.

Therefore, the sanitary product confirmation can simply occur by selecting and confirming by the push-button OK (also in this case, in order to have an uniform behaviour, as the amounts of the different preselected sanitary products are confirmed for the concerned cabinet/trolley, the symbol before the product code changes from "-" to "+" in order to identify the sanitary products already confirmed and therefore taken even though by this mode there is also an optical confirmation thanks to the selection module LEDs). Advantageously, it is possible to eliminate from the withdrawal list a product by selecting it and taking the withdrawal amount to 0.

The closure routine of the cabinet/trolley is analogous to the preceding mode, except for the confirmation of all the preselected sanitary products and contained in the cabinet/trolley, having Q_{M} > 0, which were selected and confirmed by depressing "OK".

Such condition visually corresponds to the absence of selection modules 10 having turned-on red colour LEDs.

### Withdrawal mode for cabinets/trolleys of the "open with total control" type

Such mode is mainly used in environments wherein the withdrawal quickness is determinant, and it is unknown, a priori, which sanitary products will be necessary. Such functionality is enabled by the system configuration.

The user selects, from the Workstation WKS2, the withdrawal mode OPEN.

Such state provides the opening of all the cabinets/trolleys, by eliminating the restraint of only one opened cabinet/trolley at a time of the preceding modes.

The withdrawal modes by the operator are the same as the ones described in the total control mode, with the difference that it is possible to select any product by depressing the corresponding selection module 10 and specifying the withdrawal amounts by the displaying module 11. The confirmation of the operation is as in the total control mode described before.

### Withdrawal mode of the "mixed with total control" type for cabinets/trolleys

This mode has the characteristics of the total control mode, wherein the sanitary products which should be anyway taken are predefined, and further it enables to open all the cabinets/trolleys, in an open mode, in order to possibly take further sanitary products.

The pre-selection of the withdrawal list of drugs/medical-surgical devices by software enables to highlight in red colour the sanitary products to be taken, but by the mixed mode it is possible to select any further product non comprised in the withdrawal list, by consequently signalling the used amounts.

The mixed mode is instrumental in identifying the kits for operating rooms.

Moreover, the "Smart Logistic Ward" software enables to manage the activity of the products returned from the patient, for reintegrating the discharged sanitary products (by the withdrawal activity) but not used. As before, the system provides different operative modes as a function of the configuration "conditioning" of the cabinets/trolleys.

The logistic/therapeutic system by the Interface Manager enables the integration with the MMS (Material Management System) in order to:
- obtain the history of the sanitary products (drugs and medical-surgical devices), the characteristic of each product and the updates;
- require the refilling of the sanitary products as necessary;
- unload the sanitary products on consignment, according to the batch specification and expiration date or by reading a bar code containing said information;
- electronically obtain the DDTs (transport documents) or the refilling documents of the different delivered sanitary products, for automatically refilling the sanitary products necessary to reintegrate the inventory of the different sanitary products.

There is a MMS interface for each department/U.O. (Operative Unit), and since each patient has a corresponding department/U.O. (these information being obtained from the HIS), and since each department/U.O. is associated to a cost control centre, due to the fact that each discharged product can be attributed to a corresponding patient, it is possible to obtain an optimal, objective analytical accounting for the sanitary products.

The management software, by the "Smart Logistic Ward", implements the managing functionality of the discarded sanitary products (or losses) and enables to manage dummy patients which in turn enables to work also without being connected to the network or without the HIS interface service.

Upon restoring the normal operative conditions, "Smart Logistic Ward" enables to match the dummy histories with the real histories.

The Therapy Manager software enables to manage, schedule, suspend, restore and terminate the oral medication prescriptions, i.m., e.v. for the hospitalized patients by referring to the drugs available in the pharmaceutical cabinets/trolleys of the associated department.

It is to be observed also that the management software by the Vital Parameter Manager, enables to load vital parameters, with the recording configuration and highlights possible abnormal values and possible therapeutic actions to be started.

Further, the management software with the Electronic Patient Record, implements the computerized chart of the hospitalized patient, by enabling an organic, functional gathering of the data referring to each hospitalization period; by organizing them in data incident to:
- the opening of the hospitalization;
- the administrative data;
- the initial placement of the hospitalized person;
- the treatment process;
- the scheduling of the treatment process and assistance;
- the hospitalization course;
- the end of the hospitalization;
- the attached documents.

### Examples relating to other managing activities

The management system with the "Smart Logistic Ward" implements a functionality managing the department refilling activities. The operative modes are a function of the system configuration and of the "conditioning" level of the cabinets/trolleys, according to the different above-mentioned modes.

The system automatically enables to receive the refill quantities, from the DDTs or from the refilling documents coming from the MMS, automatically uploading the refill lists belonging to the Workstations.

The management software, by the "Smart Logistic Ward", enables to manage the inventory activity. The operative modes, as in the preceding instances, are a function of the system configuration and of "conditioning" level of the cabinets/trolleys.

In the example of the total control configuration, it is possible to perform the inventory directly on the cabinet/trolley, also during a withdrawal operation, by simultaneously depressing the push-buttons "+" and "-"; after having modified the inventory quantities, by depressing again the two push-buttons, it is confirmed the new quantity.

Further, the system enables an administrative discharge (for example, a recalled batch), the relocation of the sanitary products and the management of the kits.

The kit is an optimized set of sanitary products (drugs and/or medical-surgical devices) that fulfil a clinical-sanitary necessity, such, for example, a procedure or a surgery.

The kits can be real or virtual, wherein the real kits are preformed groups of sanitary products which have a unique, specific placement, while the virtual kits are a set of sanitary products, located in different positions (different cabinets/trolleys or different placements in the same cabinet/trolley) but which can/must be quickly taken, when they are required, thanks to the system support.

### Example relating the supervision activity

The management software, by the "Smart Logistic Ward", offers a real-time tool for monitoring the handling of the sanitary products for the Central Pharmacy and the Depository in charge of arranging the stocks, by showing the expiring sanitary products and the under stock sanitary products.

The management software, by the "Smart Logistic Ward", enables, from the configuration device, the definition of the sanitary product stocks as a function of predetermined levels: rearrangement point, minimum level, maximum level, etc.

Further, the management software, by the "Smart Logistic Ward", offers a tool for completely controlling the activities on the cabinets/trolleys, tracking the accesses, the operation type and the handled quantities.

### Example relating the system configuration

Referring to the configurability, the management system, by the "Smart Logistic Ward", allows to:
a) outline the organization of the enterprise wherein it is installed by starting, for example, from the Group or Local Sanitary Unit, then passing through the Hospital Facilities and/or Health Centres, afterwards through the departments, then through the rooms containing the sanitary products (drugs/medical-surgical devices) lastly arriving to the single cabinets/trolleys;
b) define the logic organization of each cabinet/trolley, by setting the placements (the placement is defined by: position, location, depth) to which the sanitary products are associated;
c) associate and configure the hardware devices of each cabinet/trolley;
d) associate the sanitary products to the different placements by defining the rearrangement modes (constant time or constant quantity);
e) by hardware devices specified in the following:
   - the communications between the coordinator CDD and the room Workstation: both by Ethernet (TCP/IP) and by the serial line (RS232);
   - the communications with the cabinets/trolleys both wireless (passing through the coordinator CDD) according to the standard IEE 802.15.4, and wired (without passing through the coordinator CDD) by Ethernet;
   - the management of the opening module of the cabinet/trolley both by the room coordinator CDD and the end-device (EDD) of the cabinet/trolley.

What has been described ensures a high flexibility level, by adapting the system to the environments in which it is installed.

In case it is desired to condition the cabinets/trolleys by just one closing module, for example, it is better to adopt a wireless solution by one coordinator CDD (or several CDDs) directly communicating to the closing modules 16, of the cabinets/trolleys. This choice prevents from wiring the cabinets/trolleys by using, in the department, a minimally invasive solution.

In the instances in which it is not possible to use a wireless approach, due to noises to the network, etc., it is possible to use wired approaches wherein the Workstation directly communicates to the cabinet/trolley by TCP/IP.

### Examples relating other characteristic of the management system

The system is fault tolerant at several levels:
Level 1: the system automatically performs a switch between the wired network and the wireless one for ensuring the communications between the cabinets/trolleys and the room gateway in case of momentary faults/malfunctions of the hospital Ethernet.
Level 2: the presence of the local Database DB-3 enables the system to operate also when it is not possible to reach the local server.
Level 3: the presence of the local Database DB-2 enables the system to operate also when it is not possible to reach the central server DB.
Level 4: the possibility to form and manage dummy patients for avoiding eventual inefficiencies of the interfacing between the "Smart Logistic Ward" management software and the HIS.

The "Smart Logistic Ward" software enables the harmonization, that is the correct re-association of the dummy patients to the real ones.

Level 5: the management software, "Smart Logistic Ward", implements the logic deletion and not the physical one of the significant data present in the database in order to enable the retrieval of data erroneously cancelled. By using modular-type interface means such as, for example, the displaying, closing and selection modules, it is moreover possible to obtain a logistic management system at hospital departments which is complete, flexible and reliable, allowing also to preserve the existing cabinets and trolleys.

Moreover, it is to be observed that, according to the prior art, the prescription and administration of the medication are unrelated activities which by being often managed by different informative systems and performed by different sanitary operators (doctors and paramedics). Conversely, by the described management system, it is eliminated this unrelation and this system enables the doctor to prescribe only the sanitary products which are available in the department and the paramedic to distribute/administrate them as a function of the computerized medications, made by the department doctors, with the attached details regarding the administration/distribution modes and the possible alternative sanitary products.

When a patient is transferred from a department to another in the hospital, the described management system enables also the related transfer of the medication.

The same system moreover enables to organically and functionally gather data regarding single hospitalization cases (computerized chart).

## Claims

1. Logistic/therapeutic management system comprising:
at least one management computer (1, 3, WKS1) comprising at least one management software;
at least one database (DB-1, DB-2, DB-3) containing data associated to drugs and/or medical-surgical devices and patients and managed by the at least one management computer;
at least one cabinet/trolley (200B) adapted to receive drugs/devices;
interface-communications means (8; 10, 11) adapted to exchange command information/signals between one user of the at least one cabinet/trolley and the at least one computer;
and **characterized by** the fact that said interface-communications means and the management system are configured to:
communicate to the user, from the at least one management computer and the at least one database and by the interface-communications means: data indentifying a drug/device to be taken by the at least one cabinet/trolley and data indicating an amount to be taken of said drug/device associated to a patient;
communicate to the at least one management computer by the interface-communications means: data relating a withdrawal from said at least one cabinet/trolley of the drug/device performed by the user.

2. Management system according to claim 1, wherein the interface-communications means are structured in order to comprise:
a confirmation module (11) configured to enable the user to send to the at least one management computer and to the at least one database a confirmation signal indicating a performed withdrawal according to said data identifying the drug/device and indicating the amount.

3. Management system according to claim 2, said interface-communications means comprise a displaying module (11) adapted to show to the user said data identifying the drug/device and indicating said amount to be taken from the cabinet/trolley.

4. Management system according to claim 3, wherein said interface-communications means comprises a plurality of selecting modules (10) mounted on said at least one cabinet/trolley and each of them being associated to a kind of drug/device; each selecting module (10) comprising:
a selection push-button (20) activable by the user in order to show on the displaying module (11) at least one of the following data: data identifying the drug/device and data indicating the amount.

5. Management system according to claim 3, wherein said interface-communications means moreover comprises:
a modifying module (11) structured to allow the user to send to the at least one management computer a modifying signal indicating a performed withdrawal of the drug/device from the corresponding cabinet/trolley according to further taken amount data different from said data indicating the quantities to be taken.

6. Management system according to claim 1 or 7, wherein said system is moreover configured to communicate to the user, from the at least one management computer, and by the interface-communications means:
position data adapted to define the position of said drug/device to be taken from the corresponding cabinet/trolley;
updating said database as a function of the data regarding the performed withdrawal communicated by said interface-communications means.

7. Management system according to claim 6, wherein:
said position data comprises a coordinate group of the drug/device in the corresponding cabinet/trolley, organized according to a path defining: a shelf; a shelf portion; a depth;
said data identifying a drug/device and said data relating to an amount to be taken comprises: a quantity expressed in unit measure UoM; the name of a drug, a first code of a product associated to the drug.

8. Management system according to claim 1, wherein the system is configured to communicate to the user the data identifying a plurality of drugs/devices and corresponding data of amounts to be taken from said at least one cabinet/trolley and associated to the same patient.

9. Management system according to claims 6 and 8, wherein the plurality of drugs/devices comprises a first drug/device or a first portion of a drug/device arranged in a first cabinet/trolley and a second drug/device or a second portion of a said drug/device arranged in a second cabinet/trolley; said position data comprising information adapted to identify the first and second cabinet/trolley according to a withdrawal sequence.

10. Management system according to claim 1, wherein said at least one cabinet/trolley is a cabinet comprising shelves and/or drawers, closed by at least one door.

11. Management system according to claim 1, wherein said at least one cabinet/trolley is a transportable trolley provided with wheels and shelves and/or drawers, closed by one or more doors.

12. Management system according to claim 1, wherein said interface-communications means comprise at least one first interface-communications device fitted on said at least one cabinet/trolley.

13. Management system according to claim 12, wherein said interface-communications means comprises at least one second interface-communications device different from said at least one cabinet/trolley and located in the same room in which is present said at least one cabinet/trolley.

14. Management system according to claim 1, moreover comprising:
a closing module (9) associated to the at least one cabinet/trolley and to said interface-communications means and adapted to exchange data and control signals with the management computer;
an opening/closing lock (14) of said at least one cabinet/trolley controlled by said closing module.

15. Management system according to claim 9, wherein the first and second cabinets/trolleys are provided with a corresponding optical indicator (15), for example a LED, adapted to signal to the user an opening or closing condition of said first and second cabinets/trolleys.

16. Management system according to claim 1, wherein the system is configured for delivering from the user and by said interface-communications means, data indicating amounts and adapted to identify drugs/devices to be put in said at least one cabinet/trolley.

17. Management system according to claim 1, wherein said interface means are configured for enabling the exchange with the at least one computer, of further data regarding withdrawal/return/discharge/inventory operations in said at least one cabinet/trolley for drugs/medical-surgical devices.

18. Management system according to claims 4 and/or 14, wherein at least one module between said selection module (10), said displaying module (11), said closing module (9) is an apparatus removably fitted on the at least one cabinet/trolley.

19. Management system according to claim 1, wherein:
said at least one management computer comprises a plurality of computers (1, 3, WKS1);
said at least one cabinet/trolley comprises a plurality of cabinets/trolleys operatively associated to the plurality of computers and to be arranged in rooms of one or more buildings of sanitary institutions to manage the drug/medical-surgical device logistics thereof;
said database is of the distributed-type and comprises a central database (DB1) and a plurality of local databases (DB-2, DB-3) respectively associated to single Hospitals/Health Centres and to corresponding rooms containing drugs and medical-surgical devices.
